# EUROPÄISCHE PATENTSCHRIFT

(11) **EP 0 406 805 B1**
(45) Veröffentlichungstag und Bekanntmachung des Hinweises auf die Patenterteilung: **13.12.1995**
(21) Anmeldenummer: 90112691.2
(22) Anmeldetag: 03.07.1990
(51) Int. Cl.: G01N 11/08

(54) **Echtzeit-Kapillarrheometer-Anordnung**
Real time capillary rheometer arrangement
Dispositif rhéométrique à capillaire en temps réel

(30) Priorität: 03.07.1989 DE 3921841
(43) Veröffentlichungstag der Anmeldung: 09.01.1991
(73) Patentinhaber: GÖTTFERT WERKSTOFF-PRÜFMASCHINEN GMBH, D-74722 Buchen (DE)
(72) Erfinder: Gleissle, Wolfgang, Dr.-Ing., D-6729 Hagenbach (DE)
(74) Vertreter: Naumann, Ulrich, Dr.-Ing.

(56) Entgegenhaltungen:
- DE-A- 3 841 312
- DE-B- 1 801 407
- DE-U- 8 709 717
- US-A- 4 817 416

## Beschreibung

Die Erfindung betrifft eine Kapillarrheometer-Anordnung zur kontinuierlichen und diskontinuierlichen Messung der Viskosität von unter konstantem oder variierendem Druck stehenden Substanzen (Meßflüssigkeiten), insbes. Polymerschmelzen oder anderen viskoselastischen Flüssigkeiten, insbes. in einem Viskositätsbereich von 1 Pas bis 3.10⁶ Pas.

Bekannte kontinuierlich messende Kapillarrheometer (Viskosimeter) ermitteln die Druckdifferenz zwischen Eingang und Ausgang einer Meßkapillare mit bekannten geometrischen Abmessungen bei konstantem oder variablem bekanntem Meßstrom. Als Dosierpumpen werden im allgemeinen Zahnradpumpen, bei hochviskosen Substanzen bevorzugt Zahnradspinnpumpen verwendet. Im Idealfall der newtonschen Flüssigkeit ist die Druckdifferenz direkt proportional zur Viskosität. Für nicht-newtonsche Flüssigkeiten, insbes. bei viskoelastischen Flüssigkeiten wie Polymerschmelzen, müssen für die exakte Berechnung der Viskosität aus dem Volumenstrom und der Druckdifferenz des Meßvolumenstroms Kalibriermessungen durchgeführt werden. Bei diesen ist die Viskosität selbst eine Funktion der Schergeschwindigkeit bzw. bei Anwendung der Kapillarrheometrie eine Funktion des Volumenstroms V̇ durch die Meßkapillare. Bei solchen Flüssigkeiten muß für eine hinreichend genaue Charakterisierung des Fließverhaltens die Druckdifferenz über einen möglichst großen Bereich des Meßvolumenstroms gemessen werden. Dabei ist es häufig notwendig, diesen über mehrere, vielfach drei bis vier Zehnerpotenzen zu variieren. In der Regel sind dabei besonders bei hochpolymeren Flüssigkeiten die Unterschiede in der Viskosität zweier chemisch ähnlicher Stoffe bei kleinen Schergeschwindigkeiten viel größer als bei hohen Schergeschwindigkeiten.

Wenn man ein kontinuierlich arbeitendes Rheometer zur Messung der Viskosität einer Substanz in einem Reaktor (beispielsweise zur Polymerisation) zur Prozeßüberwachung oder gar zur Prozeßsteuerung einsetzen will, muß dieses in der Lage sein, einen großen Bereich des Meßvolumenstroms zu überdecken. Wenn es bei konstanter Drehzahl oder konstantem Druck arbeiten soll, wird zweckmäßigerweise bei niedriger Schergeschwindigkeit bzw. bei kleiner Schubspannung gemessen. Hierzu ist die zu messende Flüssigkeit (Meßflüssigkeit) aus dem Prozeßraum (z.B. eines Reaktors, eines Mischers oder eines Extruders) von der Probeentnahmestelle der Meßstelle über eine Leitungsverbindung dem Rheometer mit der Dosierpumpe und der Meßkapillare zuzuleiten. Durch diese wird dann die Meßflüssigkeit gefördert. Aus der Drehzahl der Dosierpumpe und der sich jeweils über die Meßkapillare einstellenden Druckdifferenz gewinnt man Punkte der zu ermittelnden Fließfunktion. Dabei sind prinzipiell zwei Versuchsführungen möglich.

Bei jeweils konstantgehaltener Dosierpumpendrehzahl (konstantem Volumenstrom) mißt man die sich jeweils einstellende Druckdifferenz (Spannversuch). Die Pumpendrehzahl wir dabei in dem für Spinnpumpen sinnvollen Bereich zwischen 0,1 Upm und 100 Upm variiert. Der Volumenstrom ist dann bei der langsamsten Drehzahl tausendmal kleiner als bei der maximalen Drehzahl. Dies ist die in der Rheometrie häufigst angewandte Versuchsführung.

Man mißt bei jeweils über der Meßkapillare konstant gehaltener Druckdifferenz. Dies ist das in der Kunststoffindustrie übliche Meßverfahren für die Fließeigenschaften von Kunststoffschmelzen.

Ein konstanter Druck auf eine Flüssigkeit kann z.B. durch ein konstantes Gewicht auf einem Prüfzylinder erzeugt werden. Bei vorgegebenen Kapillarabmessungen stellt sich eine konstante, innere Schubspannung ein (Kriechversuch). Die abhängige Meßgröße ist hier der Volumenstrom. Ein entsprechendes Meßgerät, der sogenannte Schmelzindexer, und die Meßprozedur, die Schmelzindexmessung, sind weltweit standardisiert. Der ermittelte Meßwert, der Schmelzindex, ist die pro Zeiteinheit aus der Meßkapillare ausfließende Flüssigkeitsmenge. Die Einheit des Schmelzindexes ist g/10 min bzw. cm³/10 min. Für die analoge Messung mit einer Dosierpumpe bedingt diese Vorgabe einer bestimmten konstanten Druckdifferenz eine entsprechende Nachregelung der Drehzahl der Dosierpumpe. Sie ist das Meßergebnis. Um beispielswiese Schmelzindizes viskoser Flüssigkeiten im praktisch vorkommenden Bereich von Kunststoffschmelzen bestimmen zu können, muß sich die Pumpendrehzahl im Verhältnis von bis zu 1 : 1000 ändern lassen.

Kapillarrheometer zur kontinuierlichen Messung der Viskosität von Flüssigkeiten in Reaktoren, Mischern oder Leitungen setzt man in der Weise ein, daß eine Dosierpumpe den zur Messung notwendigen Flüssigkeitsstrom dem Reaktor oder dergl. entnimmt und durch die Meßkapillare drückt. Auch hier wird die sich über deren Länge einstellende Druckdifferenz gemessen. Die Meßflüssigkeit wird entweder ins Freie entlassen (Bypass-Rheometer), oder wieder in den Reaktor oder dergl. zurückgefördert (Seitenstromrheometer). Zu beiden Bauarten stellt das Rheometer ein (in einer Linie fortlaufendes) geschlossenes System dar, dessen Gesamtdurchsatz nur von der Dosierpumpe bestimmt wird. Beim praktischen Einsatz von Kapillarrheometern im Seitenstrom von unter variierenden Drücken betriebenen Reaktoren hat sich die Druckdifferenz trotz konstant gehaltener Fördermenge wegen der Druckabhängigkeit der Viskosität als zusätzlich vom Vordruck abhängig erwiesen. Zur Ausschaltung der Vordruckabhängigkeit hat man der Meßkapillare eine zweite, die Mengenleistung der Dosierpumpe überschreitende Austragspumpe nachgeschaltet, wodurch ein konstanter Austrittsdruck aus der Meßkapillare von praktisch Null erzielt wird (US-PS 4 817 416). Auch derartige Systeme sind im Einsatz. Bei beiden Systemen, also ob mit oder ohne Austragspumpe, ist der durch das Rheometer fließende Meßflüssigkeitsstrom in der Meßkapillare und in den Zulaufleitungen gleich groß und proportional der Drehzahl der Dosierpumpe, die sich aus den angegebenen Gründen um viele Zehnerpotenzen ändern können muß. Mit der Änderung der Drehzahl bzw. des Fördervolumenstromes ändert sich umgekehrt proportional die mittlere Verweilzeit der Meßflüssigkeit in der Rheometeranordnung. Das bedeutet, daß bei einer angenommenen Drehzahlreduzierung um z.B. den Faktor 500 (100) sich die Verweilzeit um den Faktor 500 (100) erhöht. Die spezifischen Fördervolumina von Spinnpumpen liegen etwa zwischen 0,5 cm³/Upm und 3,2 cm³/Upm. Typische maximale Dauerdrehzahlen von Spinnpumpen sind etwa 100 Upm.

Bei einem bekannten Einsatz eines Seitenstrom-Kapillarrheometers befinden sich zwischen der Probenahmestelle und dem Ende der Meßkapillare etwa 40 cm³ Meßflüssigkeit. Die Dosierpumpe fördert 0,65 cm³/Upm. Um bei konstanter Druckdifferenz den Bereich der üblicherweise auftretenden Schmelzindizes (DIN 53 735;0,1 ≦ MFI ≦ 50) abdecken zu können, muß die Drehzahl mindestens zwischen 100 Upm und 0,2 Upm variiert werden. Dies würde zu mittleren Verweilzeiten von etwa 37 Sekunden bis über 5 Stunden führen, was nicht nur zeigt, daß dieser Bereich nicht beherrscht werden kann, sondern auch, daß selbst bei engeren Bereichen Variation des Schmelzindex während des Prozesses oder der Reaktion eine kontinuierliche Prozeßsteuerung nicht oder nur höchst ungenau möglich ist. Ein Meßergebnis darf nicht erst lange nach der Probenahme vorliegen. Zudem sind Polymerschmelzen thermisch häufig nicht stabil genug, um eine sehr lange Zeit hohe Prozeßtemperaturen ohne molekulare Änderungen zu ertragen (thermische Degradation).

Um wenigstens bei nicht zu großen Viskositätsänderungen zu einigermaßen brauchbaren Ergnissen zu kommen, hat man die Transportvolumina bis zur Meßkapillare möglichst gering gewählt, beispielsweise durch direkte Montage des Kapillarrheometers am Reaktor oder dergl.. Diesem Aneinanderrücken von Probenahmestelle und Rheometer sind aber schnell natürliche Grenzen aufgrund der Schwierigkeiten mit der Handhabung und/oder mit der Kühlung bzw. Heizung des Rheometers verbunden. Es hat sich gezeigt, daß das Ansaugvolumen vor der Dosierpumpe immer noch um mindestens das Zehnfache größer ist als das Volumen der Meßkapillare einschließlich des Volumens der Zulaufkanäle der Dosierpumpe (2 bis 5 cm³). Die Durchströmzeit durch diesen notwendigen Teil der Rheometeranordnung ist damit zehnmal länger als die tatsächliche Meßzeit während der Durchströmung der Kapillare. Dennoch läßt sich dieses niedrige Verhältnis nur durch optimierte Konstruktion des Rheometers und unmittelbare Montage an der Probenahmestelle des Reaktors oder dergl. erreichen. Im allgemeinen erlauben es die tatsächlich vorliegenden Umstände nicht ein so niedriges Verhältnis einzuhalten. Das bedeutet, daß die Ansprechzeit des kontinuierlich arbeitenden Rheometers im wesentlichen durch die Zuleitung und nicht durch das Rheometer bestimmt ist.

Bei einer newtonschen Flüssigkeit ist die Schergeschwindigkeit bei gleichem Volumenstrom umgekehrt proportional zur dritten Potenz des Kapillardurchmessers. Neben der Minimierung des Volumens der Zulaufleitungen ist die Erweiterung des Durchmessers der Meßkapillare eine bekannte Methode, um Verweilzeiten bei kleinen Schergeschwindigkeiten genügend kurz zu halten. Dies bedeutet aber, daß große Schmelzindizes nicht mehr gemessen werden können, da dann erstens die zulässigen Höchstdrehzahlen der Dosierpumpen überschritten werden und zweitens die durch die Pumpe in die Meßflüssigkeit eingetretene Energie zu unzulässig großen Temperaturerhöhungen führt. Um den gleichen Meßbereich zu erhalten, müssen daher Meßkapillaren mit unterschiedlichem Durchmesser eingesetzt werden. Ein Austausch einer Meßkapillare gegen eine Meßkapillare mit anderem Durchmesser ist während der Kontrolle eines Prozesses nicht möglich, weil die Umrüstzeiten bei den bekannten Konstruktionen (mehrere) Stunden betragen und das Rheometer bei nicht newtonschen Stoffen neu kalibriert werden müßte.

Der Erfindung liegt die Aufgabe zugrunde, eine Echtzeit-Kapillarrheometer-Anordnung zur kontinuierlichen oder diskontinuierlichen Messung der Viskosität von flüssigen Substanzen, insbesondere Polymerschmelzen u.ä., anzugeben, die sich unabhängig von den Gegebenheiten im Bereich der Probenahmestelle des Reaktors, des Extruders, der Leitung oder dergl. einsetzen läßt und einen großen Viskositätsbereich, insbes. auch während einer Reaktion, ohne Umrüstung schnell und genau abzudecken vermag.

Eine diese Aufgabe lösende Kapillarrheometer-Anordnung ist in den Patentansprüchen 1,2,6 und 7 und hinsichtlich ihrer Ausgestaltungen in den Unteransprüchen gekennzeichnet.

Dieses Ziel wird erfindungsgemäß durch eine Kapillarrheometer-Anordnung erreicht, bei der durch Parallelschaltung (Überlagerung) eines weiteren Flüssigkeitsstromes (zum eigentlichen Meßflüssigkeitsstrom) die Zufuhr von Flüssigkeit zur Dosierpumpe nicht durch die Größe des Meßflüssigkeitsstromes selbst begrenzt wird sondern unabhängig von der momentanen Größe des Meßflüssigkeitsstromes für einen schnellen Austausch der Meßflüssigkeit in den Zulaufkanälen zur Dosierpumpe sorgt. Der Zufuhrvolumenstrom kann durch eine Zufuhrpumpe mit großer spezifischer Förderleistung bzw. mit genügend hoher Drehzahl immer so hoch gehalten werden, daß die Verzögerungszeit zwischen Probenahme und Messung immer im Bereich weniger Sekunden bleibt. Der von der Dosierpumpe nicht benötigte Flüssigkeitsvolumenstrom wird durch eine Seitenleitung an der Meßkapillare vorbeigeführt. Unabhängig von der Drehzahl der Dosierpumpe steht die zu prüfende Flüssigkeit nach kurzer Zeit zur Messung zur Verfügung. Bei kleinen Fördervolumenströmen der Dosierpumpe kann nach der erfindungsgemäßen Anordnung nun die Ansprechzeit um etwa das Verhältnis des Meßvolumens zum Volumen aller Zulaufkanäle und -leitungen verkürzt werden. Diese Verkürzung erfolgt um mindestens den Faktor 10 gegenüber üblichen Meßgegebenheiten. Dabei bleibt die Kapillarrheometer-Anordnung über einen großen Meßbereich ständig meßbereit, ohne daß bei zu großen Änderungen der Viskosität der Meßflüssigkeit mehrere Stunden Umrüstzeit erforderlich werden.

Die mit einer zusätzlichen Zufuhrpumpe versehene kontinuierlich messende Kapillarrheometer-Anordnung hat gegenüber den bekannten Anordnungen, deren Förderstrom nur durch die Dosierpumpe, und gegebenenfalls die eine vorgesehene Austragspumpe, bestimmt wird, erhebliche Vorteile.

Es sind Messungen bei sehr kleinen Schergeschwindigkeiten, also in einem Bereich, in dem sich vor allem Polymerschmelzen und Lösungen stark unterscheiden, auch bei thermischer Empfindlichkeit möglich, ohne daß unzulässig lange Verweilzeiten, die zu thermischer Degradation führen können, auftreten.

Auch bei kleinen Schergeschwindigkeiten lassen sich noch Messungen zur Qualitätskontrolle durchführen, ohne daß unzulässig große Verzögerungszeiten auftreten.

Es können kontinuierlich messende Kapillarrheometer zur Ermitlung von Fließkurven über einen großen Bereich von Schergeschwindigkeiten (mehrere Zehnerpotenzen) eingesetzt werden, ohne daß in einem notwendig breiten Meßbereich unzulässig große Totzeiten auftreten.

Kontinuierlich messende Kapillarrheometer lassen sich auch zur kontinuierlichen Prozeßsteuerung im Bereich kleiner Schergeschwindigkeiten einsetzen, ohne daß für die Prozeßsteuerung unzulässig lange Totzeiten auftreten.

Die von dem Meßvolumenstrom unabhängige Produktzuführung gestattet eine freizügige Konstruktion der Zuleitung zum Rheometer. Häufig sind die möglichen Probenahmestellen an Reaktoren, Extrudern, Rohrleitungen und daher sehr unzugänglich, so daß relativ lange Zuleitungen zum Rheometer unumgänglich sind. Gerade für solche Fälle kann die Verweilzeit im Gesamtmeßsystem - Zulaufkanal und Meßkapillare - häufig erst durch eine erfindungsgemäße Rheometeranordnung so klein gehalten werden, daß Messungen überhaupt möglich sind.

Bei Messungen mit konstant gehaltener Druckdifferenz können Polymerschmelzen über mehrere Zehnerpotenzen des Volumenfließindexes gemessen werden, ohne daß dabei große Verweilzeiten im Gesamtsystem in Kauf genommen werden müssen.

Durch die Erfindung wird auch das Problem prozeßbedingter schnell schwankender Temperaturen bei der Prozeßführung erstmals für viele Fälle hinreichend gelöst. Die Viskosität fast aller Stoffe ist stark temperatur- und scherspannungsabhängig. Bei den meisten Stoffen und Mischungen, für deren Viskositätsbestimmung Kapillarrheometer eingesetzt werden, insbes. bei Polymerschmelzen in Reaktoren, ist so ungünstig, daß auch nur annäherende Temperaturkonstanz praktisch nicht durch Temperierung erreichbar ist. Rechnerische Korrekturen der Messung von einer gemessenen Temperatur und bekannten Schergeschwindigkeit auf eine Bezugstemperatur sind i.a. nicht möglich oder viel zu aufwendig und/oder langwierig, um praktisch infrage zu kommen (variabel sind Temperatur, Schergeschwindigkeit, Polyimerisationsgrad u.a.). Durch die Erfindung steht ein vom Meßflüssigkeitsvolumenstrom unabhängiger Flüssigkeitsvolumenstrom zur Verfügung, der unabhängig und gegebenenfalls auch genauer temperierbar ist. Eine unabhängig vom Meßzustand des Rheometers regelbare Temperierstrecke (Heizmengenstrom oder Volumenstrom reguliert) bietet alle Vorteile der schnellen Zuführung der Meßflüssigkeit zum Rheometer, der Minimierung der Verzögerungszeit der Viskositätsmessung als auch der optimalen und schnellen Einstellung der Solltemperatur der Meßflüssigkeit.

Ausführungsbeispiele der erfindungsgemäßen Kapillarrheometer-Anordnung sind anhand einer Zeichnung näher erläutert, in der zeigt:
- Fig. 1: eine erste Ausführungsform mit einer Seitenstromanordnung,
- Fig. 2: eine Abwandlung der ersten Ausführungsform mit einer Seitenstromanordnung,
- Fig. 3: eine zweite Ausführungsform mit einer Seitenstromanordnung,
- Fig. 4: eine dritte Ausführungsform mit einer Seitenstromanordnung,
- Fig. 5: eine Abwandlung der dritten Ausführungsform mit einer Seitenstromanordnung,
- Fig. 6: eine vierte Ausführungsform mit einer Seitenstromananordnung, und
- Fig. 7: eine fünfte Ausführungsform mit einer Bypass-Anordnung.

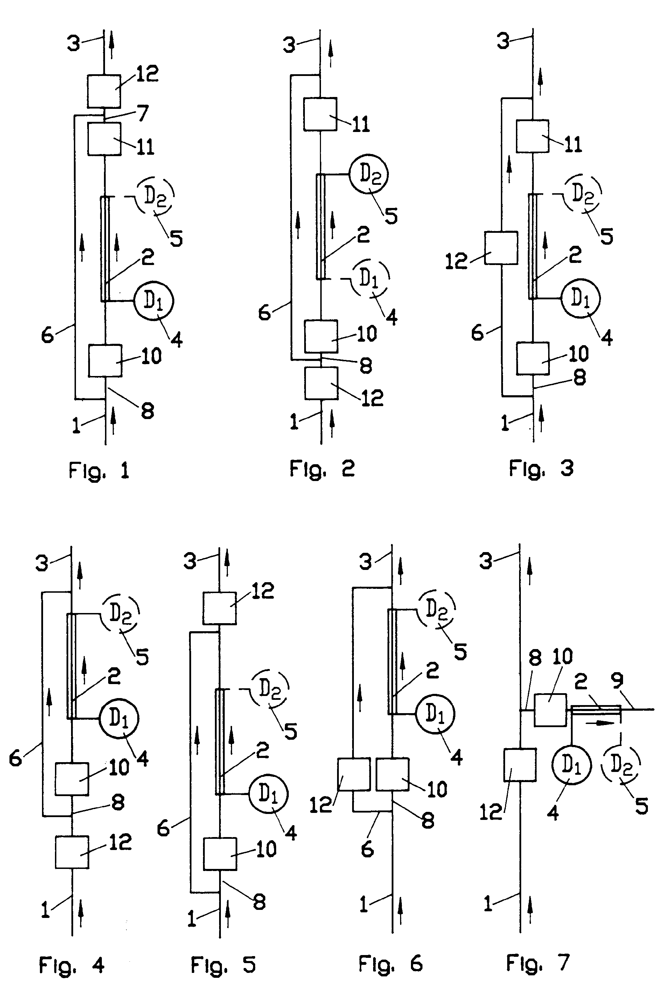

Bei den Ausführungsformen nach den Figuren 1 bis 6 wird die der Probeentnahmestelle eines nicht dargestellten Reaktors entnommene Meßflüssigkeit über eine Zulaufleitung 1 und eine Dosierpumpe 10 einer Meßkapillare 2 zugeführt, aus der sie mittels einer Austragspumpe 11 und über eine Zufuhrpumpe 12 in eine Austragsleitung 3 ausgetragen wird. Zur Bestimmung der Durckdifferenz längs der Meßkapillare 2 befindet sich an deren Einlaß ein Druckmeßfühler 4 und an dessen Auslaß ein Druckmeßfühler 5. Eine Seitenleitung 6 ist an die Zulaufleitung 1 vor dem Einlaß in die Dosierpumpe 10 angeschlossen und führt in eine die Austragspumpe 11 mit der Zufuhrpumpe 12 verbindende Verbindungsleitung 7. Auf diese Weise ist eine Seitenstromanordnung geschaffen. Wegen der Eliminierung des Einflusses des Druckes im Reaktor auf die Messung der Viskosität in der Meßkapillare 2 wurden zwei Pumpen vorgesehen. Neben der Dosierpumpe 10 hat die Austragspumpe 11 die Aufgabe, die Meßkapillare 3 gegen den Druck im Reaktor abzudichten. Die Austragspumpe 11 hat üblicherweise eine geringfügig größere Förderleistung als die Dosierpumpe 10. Stets soll die Verbindungsstrecke 8 zwischen der Entnahmestelle des Meßvolumenstroms aus der Zufuhrleitung 1 bzw. der Seitenleitung 6 und dem Rheometer möglichst kurz sein.

Eine in Fig. 2 dargestellte Abwandlung nach Anspruch 3 sieht vor, daß die Zufuhrpumpe 12 der Dosierpumpe 10 vorgeschaltet und die Seitenleitung 6 einlaßseitig an die Verbindung zwischen diesen beiden Pumpen angeschlossen ist.

Die Meßkapillare 2 hat übliche Abmessungen. Bei Rundlochkapillaren liegen der Durchmesser zwischen 0,1 mm und 8 mm und die Länge vorteilhaft zwischen 5 mm und 100 mm. Es können auch Schlitzkapillaren eingesetzt werden, wobei die Breite der Schlitze 5 mm bis 20 mm und deren Tiefe 0,2 mm bis 4 mm betragen kann. Beide Kapillartypen sind auswechselbar. Wenn eine Austragspumpe 11 mit geringfügig größerer Förderleistung als die Dospierpumpe benutzt wird, ist eine Messung des Drucks am Auslaß der Meßkapillare nicht notwendig, da der Druck vor der Austragspumpe vernachlässigbar klein ist. Durch die hier verwirklichte Reihenschaltung ist erreicht, daß der Volumenstrom in der Zufuhrleitung 1 und in der Austragsleitung 3 unabhängig von der Fördermenge der Dosierpumpe 10 konstant groß ist. Die Drehrichtung der Pumpe ist frei wählbar, d.h. die Zufuhrpumpe 12 kann entweder hinter der Austragspumpe 11, sh. Fig. 1, oder vor die Dosierpumpe 10 in sinngemäßer Drehrichtung geschaltet werden, sh. Fig. 2.

Eine zweite vorteilhafte Ausführungsform ist in Fig. 3 dargestellt, bei welcher die Zufuhrpumpe 12 in der Seitenleitung 6 vorgesehen ist. Bei dieser Anordnung erhält man bei konstanter Drehzahl der Zufuhrpumpe 12 einen minimalen Gesamtförderstrom, wenn die Dosierpumpe und die Austragspumpe 11 jeweils geringste Drehzahl haben. Der Gesamtförderstrom ist dann am höchsten, wenn die Messung bei maximalen Schergeschwindigkeiten durchgeführt wird. Dabei wird dann eine extrem kurze Ansprechzeit erreicht. Durch eine entsprechende proportionale Drehzahlregelung der Zufuhrpumpe 12 zu den Drehzahlen der Dosierpumpe 10 und der Austragspumpe 11 kann auch bei dieser Anordnung ein konstanter Gesamtstrom erreicht werden. Es sind wahlweise ein oder mehrere Druckmeßfühler vorsehbar.

Bei einigen Aufgabenstellungen der kontinuierichen Viskositätsmessung kann auf eine vom Prozeßdruck unabhängige Messung verzichtet werden, weil entweder die Meßflüssigkeit eine Viskosität hat, die nicht vom Druck abhängt, oder weil man die Viskosität bei dem augenblicklichen Prozeßdruck erfassen möchte. In solchen Fällen kann auf die Austragspumpe 11 verzichtet werden. Die Meßkapillare 2 ist dann gegenüber dem Prozeßraum auf einer Seite offen. Es ist in der Regel dann vorteilhaft, mit zwei Druckmeßfühlern 4 und 5 eine Druckdifferenz zur Viskositätsberechnung zu messen. Die Fig. 4 bis 6 zeigen solche vorteilhafte Anordnungen von Dosierpumpe 10 und Zufuhrpumpe 12 mit Bezug auf die Meßkapillare 2.

In Fig. 4 ist die Anordnung der Zufuhrpumpe 12 vor der Dosierpumpe 10 und vor dem Einlaß in die Seitenleitung 6 dargestellt. Diese dritte Ausführungsform ergibt einen konstanten Gesamtstrom entsprechend Fig. 1 und damit immer gleiche Verweilzeiten im Gesamtsystem.

Die in Fig. 5 dargestellte Abwandlung dieser Ausführungsform sieht vor, daß die Zufuhrpumpe 12 der Meßkapillare 2 und dem Auslaß der Seitenleitung 6 nachgeschaltet ist, die Meßflüssigkeit also ansaugt.

Fig. 6 zeigt die Parallelschaltung der Dosierpumpe 10 und der in der Seitenleitung 6 vorgesehenen Zufuhrpumpe 12. Diese vierte Ausführungsform liefert kürzestmögliche Verzögerungszeiten bei der kontinuierlichen Viskositätsmessung gegenüber den Vorgängen im Prozeßraum. Ein vorteilhafter konstanter Gesamtvolumenstrom kann durch Drehzahlregelung der Zufuhrpumpe 12 erreicht werden.

Eine weitere vorteilhafte Anordnung mit einer Zufuhrpumpe 12 zur Verringerung der Ansprechzeit des Kapillarrheometers zeigt Fig. 7. Bei dieser fünften Ausführungsform ist eine Bypass-Anordnung vorgesehen. In vielen Aufgabenstellungen ist es nicht notwendig oder auch nicht möglich, die durch die Meßkapillare 2 geflossene Meßflüssigkeit wieder zurück in den Prozeß (Reaktor, Extruder, Leitung, Mischer oder dergl.) zu fördern. Die Meßflüssigkeit muß hinter der Meßkapillare durch eine Abfuhrleitung 9 ins Freie geleitet werden. Die Meßflüssigkeit ist damit häufig verloren. Solche Rheometer sind als Bypass-Rheometer an sich bekannt. In diesem Fall dient die Zufuhrpumpe 12 nur zur schnellen Meßflüssigkeitsförderung zur Dosierpumpe 10. Der von dieser nicht benötigte Volumenstrom kann in den Prozeßraum zurückgeleitet werden. So kann auch ein kontinuierlich messendes Bypass-Rheometer bei kleinen Volumenströmen, d.h. bei vorteilhaften hohen Auflösungen der Viskosität und bei kleinen Flüssigkeitsverlusten mit trotzdem kurzen Ansprechzeiten arbeiten.

## Patentansprüche

1. Kapillarrheometer-Anordnung zur kontinuierlichen oder diskontinuierlichen Messung der Viskosität von flüssigen Substanzen (Meßflüssigkeiten), insbes. von Polymerschmelzen und anderen viskoselastischen Flüssigkeiten, mit einem Rheometer, das eine Meßkapillare (2), eine der Meßkapillare vorgeschaltete, zu einer Flüssigkeitsprobennahmestelle führende Zulaufleitung (1), eine der Meßkapillare nachgeschaltete Austragsleitung (3) für Meßflüssigkeit, eine den Meßflüssigkeitsvolumenstrom der Meßkapillare zufördernde Dosierpumpe (10) in der Zulaufleitung und eine an die Meßkapillare angeschlossene Austragspumpe (11) in der Austragsleitung (3) aufweist,
**gekennzeichnet** durch
eine Seitenleitung (6), die einlaßseitig an die zur Dosierpumpe (10) führende Zulaufleitung (1) und auslaßseitig an die an die Meßkapillare (2) angeschlossene Austragsleitung (3) angeschlossen ist und
eine Zufuhrpumpe (12), mit der Meßflüssigkeit von der Zulaufleitung (1) durch die Seitenleitung (6) zur Austragsleitung (3) gefördert wird (Fig. 1 - 3).

2. Kapillarrheometer-Anordnung zur kontinuierlichen oder diskontinuierlichen Messung der Viskosität von flüssigen Substanzen (Meßflüssigkeiten), insbes. von Polymerschmelzen und anderen viskoselastischen Flüssigkeiten, mit einem Rheometer, das eine Meßkapillare (2), eine der Meßkapillare vorgeschaltete, zu einer Flüssigkeitsprobennahmestelle führende Zulaufleitung (1), eine der Meßkapillare nachgeschaltete Austragsleitung (3) für Meßflüssigkeit und eine den Meßflüssigkeitsvolumenstrom der Meßkapillare zufördernde Dosierpumpe (10) in der Zulaufleitung aufweist,
**gekennzeichnet** durch
eine Seitenleitung (6), die einlaßseitig an die zur Dosierpumpe (10) führende Zulaufleitung (1) und auslaßseitig an die an die Meßkapillare (2) angeschlossene Austragsleitung (3) angeschlossen ist, und
eine Zufuhrpumpe (12), mit der Meßflüssigkeit von der Zulaufleitung (1) durch die Seitenleitung (6) zur Austragsleitung (3) gefördert wird (Fig. 4 - 6).

3. Kapillarrheometer-Anordnung nach Anspsruch 1 oder 2,
dadurch **gekennzeichnet**,
daß die Zufuhrpumpe (12) in der Austragsleitung (1) nach der Anschlußstelle der Seitenleitung (6) angeordnet ist (Fig. 1 und 5).

4. Kapillarrheometer-Anordnung nach Anspsruch 1 oder 2,
dadurch **gekennzeichnet**,
daß die Zufuhrpumpe (12) in der Zufuhrleitung (3) vor der Anschlußstelle der Seitenleitung (6) angeordnet ist (Fig. 2 und 4).

5. Kapillarrheometer-Anordnung nach Anspsruch 1 oder 2,
dadurch **gekennzeichnet**,
daß die Zufuhrpumpe (12) in der Seitenleitung (6) angeordnet ist (Fig. 3 und 6).

6. Kapillarrheometer-Anordnung zur kontinuierlichen oder diskontinuierlichen Messung der Viskosität von flüssigen Substanzen (Meßflüssigkeiten), insbes. von Polymerschmelzen und anderen viskoselastischen Flüssigkeiten, mit einem Rheometer, das eine Meßkapillare (2), eine der Meßkapillare vorgeschaltete, zu einer Flüssigkeitsprobennahmestelle führende Zulaufleitung (1), eine der Meßkapillare nachgeschaltete, auslaßseitig offene Meßflüssigkeits-Abfuhrleitung (9) und eine der Meßkapillare unmittelbar vorgeschaltete, ihr den Meßflüssigkeitsvolumenstrom zufördernde Dosierpumpe (10) in der Zulaufleitung (1) aufweist,
**gekennzeichnet** durch
eine an die Anschlußstelle der Dosierpumpe (10) an die Zufuhrleitung (1) angeschlossene Austragsleitung (3) und eine in der Zufuhrleitung vorgesehene Zufuhrpumpe (12) für Meßflüssigkeit (Fig. 7).

7. Kapillarrheometer-Anordnung zur kontinuierlichen oder diskontinuierlichen Messung der Viskosität von flüssigen Substanzen (Meßflüssigkeiten), insbes. von Polymerschmelzen und anderen viskoselastischen Flüssigkeiten, mit einem Rheometer, das eine Meßkapillare (2), eine der Meßkapillare vorgeschaltete, zu einer Flüssigkeitsprobennahmestelle führende Zulaufleitung (1), eine der Meßkapillare nachgeschaltete, auslaßseitig offene Meßflüssigkeits-Abfuhrleitung (9) und eine der Meßkapillare unmittelbar vorgeschaltete, ihr den Meßflüssigkeitsvolumenstrom zufördernde Dosierpumpe (10) in der Zulaufleitung (1) aufweist,
**gekennzeichnet** durch
eine an die Anschlußstelle der Dosierpumpe (10) an die Zufuhrleitung (1) angeschlossene Austragsleitung (3) und eine in der Austragsleitung (3) vorgesehene Zufuhrpumpe (12) (Abwandlung von Fig. 7).

8. Kapillarrheometer-Anordnung nach einem der Ansprüche 1 bis 7,
dadurch **gekennzeichnet**,
daß nur auf der Einlaßseite der Meßkapillare (2) ein Druckmeßfühler (4) vorgesehen ist.

9. Kapillarrheometer-Anordnung nach einem der Ansprüche 1 bis 8,
dadurch **gekennzeichnet**,
daß die Dosierpumpe (10), die Zufuhrpumpe (12) und die Austragspumpe (11) eine konstruktive Einheit bilden.

10. Kapillarrheometer-Anordnung nach Anspruch 9,
dadurch **gekennzeichnet**,
daß die Dosierpumpe (10), die Zufuhrpumpe (12) und die Austragspumpe (11) lösbar zur konstruktiven Einheit miteinander verbunden sind.

## Claims

1. Capillary rheometer arrangement for the continous or discontinuous measurement of the viscosity of fluid substances (measurement fluids), in particular of polymer melts and other viscoelastic fluids, having a rheometer which has a measuring capillary (2), a supply line (1) connected upstream of the measuring capillary and leading to a fluid sampling point, a discharge line (3) for measurement fluid, which discharge line is connected downstream of the measuring capillary, a metering pump (10) in the supply line, which metering pump conveys the measurement fluid volume flow to the measuring capillary, and a discharge pump (11) in the discharge line (3), which discharge pump is connected to the measuring capillary,
**characterized by**
a branch line (6) which is connected, on the inlet side, to the supply line (1) leading to the metering pump (10) and, on the outlet side, to the discharge line (3) connected to the measuring capillary (2), and
a feed pump (12), by means of which the measurement fluid is conveyed from the supply line (1) through the branch line (6) to the discharge line (3) (Figures 1 - 3).

2. Capillary rheometer arrangement for the continous or discontinuous measurement of the viscosity of fluid substances (measurement fluids), in particular of polymer melts and other viscoelastic fluids, having a rheometer which has a measuring capillary (2), a supply line (1) connected upstream of the measuring capillary and leading to a fluid sampling point, a discharge line (3) for measurement fluid, which discharge line is connected downstream of the measuring capillary, and a metering pump (10) in the supply line, which metering pump conveys the measurement fluid volume flow to the measuring capillary,
**characterized by**
a branch line (6) which is connected, on the inlet side, to the supply line (1) leading to the metering pump (10) and, on the outlet side, to the discharge line (3) connected to the measuring capillary (2), and
a feed pump (12), by means of which the measurement fluid is conveyed from the supply line (1) through the branch line (6) to the discharge line (3) (Figures 4 - 6).

3. Capillary rheometer arrangement according to Claim 1 or 2,
**characterized in that**
the feed pump (12) is arranged in the discharge line (3) downstream of the point of connection of the branch line (6) (Figures 1 and 5).

4. Capillary rheometer arrangement according to Claim 1 or 2,
**characterized in that**
the feed pump (12) is arranged in the supply line (1) upstream of the point of connection of the branch line (6) (Figures 2 and 4).

5. Capillary rheometer arrangement according to Claim 1 or 2,
**characterized in that**
the feed pump (12) is arranged in the branch line (6) (Figures 3 and 6).

6. Capillary rheometer arrangement for the continous or discontinuous measurement of the viscosity of fluid substances (measurement fluids), in particular of polymer melts and other viscoelastic fluids, having a rheometer which has a measuring capillary (2), a supply line (1) connected upstream of the measuring capillary and leading to a fluid sampling point, a measurement fluid evacuation line (9) connected downstream of the measuring capillary and open on the outlet side, and a metering pump (10) in the supply line (1), which metering pump is connected immediately upstream of the measuring capillary and conveys the measurement fluid volume flow to the said measuring capillary,
**characterized by**
a discharge line (3) connected to the point of connection of the metering pump (10) to the supply line (1) and a feed pump (12) for measurement fluid, which feed pump is provided in the supply line (Figure 7).

7. Capillary rheometer arrangement for the continous or discontinuous measurement of the viscosity of fluid substances (measurement fluids), in particular of polymer melts and other viscoelastic fluids, having a rheometer which has a measuring capillary (2), a supply line (1) connected upstream of the measuring capillary and leading to a fluid sampling point, a measurement fluid evacuation line (9) connected downstream of the measuring capillary and open on the outlet side, and a metering pump (10) in the supply line (1), which metering pump is connected immediately upstream of the measuring capillary and conveys the measurement fluid volume flow to the said measuring capillary,
**characterized by**
a discharge line (3) connected to the point of connection of the metering pump (10) to the supply line (1) and a feed pump (12) provided in the discharge line (3) (variation of Figure 7).

8. Capillary rheometer arrangement according to one of Claims 1 to 7,
**characterized in that**
a pressure-measuring sensor (4) is provided only on the inlet side of the measuring capillary (2).

9. Capillary rheometer arrangement according to one of Claims 1 to 8,
**characterized in that**
the metering pump (10), the feed pump (12) and the discharge pump (11) form one constructional unit.

10. Capillary rheometer arrangement according to Claim 9,
**characterized in that**
the metering pump (10), the feed pump (12) and the discharge pump (11) are connected to one another detachably to form the constructional unit.

## Revendications

1. Dispositif rhéométrique à capillaire pour la mesure en continu ou en discontinu de la viscosité de substances liquides (liquides de mesure), notamment de masses fondues de polymères et d'autres liquides à élasticité visqueuse, avec un rhéomètre qui présente un capillaire de mesure (2), une conduite d'alimentation (1) en amont du capillaire de mesure aboutissant à un poste de prélèvement d'échantillon de liquide, une conduite d'évacuation (3) pour le liquide de mesure en aval du capillaire de mesure, une pompe de dosage (10) dans la conduite d'alimentation, pompe qui produit l'alimentation du volume de liquide de mesure au capillaire de mesure et une pompe d'évacuation (11) dans la conduite d'évacuation (3), pompe qui est raccordée au capillaire de mesure,
**caractérisé**
par une conduite latérale (6) qui côté admission est raccordée à la conduite d'alimentation (1) aboutissant à la pompe de dosage (10) et qui côté sortie est raccordée à la conduite d'évacuation (3) raccordée au capillaire de mesure, et
par une pompe d'alimentation (12) à l'aide de laquelle du liquide de mesure est transporté depuis la conduite d'alimentation (1) au travers la conduite latérale (6) vers la conduite d'évacuation (Figs. 1 - 3).

2. Dispositif rhéométrique à capillaire pour la mesure en continu ou en discontinu de la viscosité de substances liquides (liquides de mesure), notamment de masses fondues de polymères et d'autres liquides à élasticité visqueuse, avec un rhéomètre qui présente un capillaire de mesure (2), une conduite d'alimentation (1) en amont du capillaire de mesure aboutissant à un poste de prélèvement d'échantillon de liquide, une conduite d'évacuation (3) pour le liquide de mesure en aval du capillaire de mesure, une pompe de dosage (10) dans la conduite d'alimentation, pompe qui produit l'alimentation du volume de liquide de mesure au capillaire de mesure,
**caractérisé**
par une conduite latérale (6) qui côté admission est raccordée à la conduite d'alimentation (1) aboutissant à la pompe de dosage (10) et qui côté sortie est raccordée à la conduite d'évacuation (3) raccordée au capillaire de mesure, et
par une pompe d'alimentation (12) à l'aide de laquelle du liquide de mesure est transporté depuis la conduite d'alimentation (1) au travers la conduite latérale (6) vers la conduite d'évacuation (Figs. 4 - 6).

3. Dispositif rhéométrique à capillaire suivant la revendication 1 ou la revendication 2,
**caractérisée**
en ce que la pompe d'alimentation (12) dans la conduite d'évacuation (1) est disposée en aval du point de raccordement de la conduite latérale (Figs. 1 et 5).

4. Dispositif rhéométrique à capillaire suivant la revendication 1 ou la revendication 2,
**caractérisé**
en ce que la pompe d'alimentation (12) dans la conduite d'évacuation (1) est disposée en amont du point de raccordement de la conduite latérale (Figs. 2 et 4).

5. Dispositif rhéométrique à capillaire suivant la revendication 1 ou la revendication 2,
**caractérisé**
en ce que la pompe d'alimentation (12) est disposée dans la conduite latérale (6) (Figs. 3 et 6).

6. Dispositif rhéométrique à capillaire pour la mesure en continu ou en discontinu de la viscosité de substances liquides (liquides de mesure), notamment de masses fondues de polymères et d'autres liquides à élasticité visqueuse, avec un rhéomètre qui présente un capillaire de mesure (2), une conduite d'alimentation (1) en amont du capillaire de mesure aboutissant à un poste de prélèvement d'échantillon de liquide, une conduite d'évacuation (9) ouverte côté évacuation, raccordée derrière le capillaire de mesure et une pompe de dosage (10) dans la conduite d'alimentation (1), pompe directement en amont du capillaire de mesure et transportant le courant de liquide de mesure,
**caractérisé**
par une conduite d'évacuation (3) raccordée au point de connexion de la pompe de dosage (10) et de la conduite d'alimentation (1) et par une pompe d'alimentation (12) pour le liquide de mesure, pompe prévue dans la conduite d'alimentation (Fig. 7).

7. Dispositif rhéométrique à capillaire pour la mesure en continu ou en discontinu de la viscosité de substances liquides (liquides de mesure), notamment de masses fondues de polymères et d'autres liquides à élasticité visqueuse, avec un rhéomètre qui présente un capillaire de mesure (2), une conduite d'alimentation (1) en amont du capillaire de mesure aboutissant à un poste de prélèvement d'échantillon de liquide, une conduite d'évacuation (9) ouverte côté évacuation, raccordée derrière le capillaire de mesure et une pompe de dosage (10) dans la conduite d'alimentation (1), pompe directement en amont du capillaire de mesure et transportant le courant de liquide de mesure,
**caractérisé**
par une conduite d'évacuation (3) raccordée au point de connexion de la pompe de dosage (10) et de la conduite d'alimentation (1) et par une pompe d'alimentation (12) prévue dans la conduite d'évacuation (3) (Variante de la Fig. 7).

8. Dispositif rhéométrique à capillaire suivant l'une quelconque des revendications de 1 à 7,
**caractérisé**
en ce que seul du côté admission du capillaire de mesure (2) est prévu un capteur de pression (4).

9. Dispositif rhéométrique à capillaire suivant l'une quelconque des revendications de 1 à 8,
**caractérisé**
en ce que la pompe de dosage (10), la pompe d'alimentation (12) et la pompe d'évacuation (11) constituent une unité de construction.

10. Dispositif rhéométrique à capillaire suivant la revendication 9,
**caractérisé**
en ce que la pompe de dosage (10), la pompe d'alimentation (12) et la pompe d'évacuation (11) sont réunies de manière amovible en une unité de construction.
